# EUROPÄISCHE PATENTANMELDUNG

(11) **EP 3 620 141 A1**
(43) Veröffentlichungstag der Anmeldung: **11.03.2020**
(21) Anmeldenummer: 18192693.2
(22) Anmeldetag: 05.09.2018
(51) Int. Cl.: A61F 13/00, A61N 5/00, A61F 7/00, A61F 7/02, A61N 5/06

(54) **VORRICHTUNG ZUR BEHANDLUNG VON JUCKREIZ UND HERPESERKRANKUNGEN MIT EINEM KONTAKTSENSOR**

(71) Anmelder: Dermapharm AG, 82031 Grünwald (DE)
(72) Erfinder: Bünger von Wurmb, Daniel, 41063 Mönchengladbach (DE)
(74) Vertreter: Hertin und Partner Rechts- und Patentanwälte PartG mbB

(57) **Zusammenfassung**

Die Erfindung betrifft eine Vorrichtung zur Behandlung von Juckreiz und/oder Herpes auf einer Haut, wobei eine Regelvorrichtung konfiguriert ist, eine Behandlungsfläche auf einer der Haut zugewandten Außenseite durch Erhitzen mindestens eines Heizelementes in einer Aufheizphase auf eine Kontakttemperatur während eines Kontaktes der Behandlungsfläche mit der Haut zu regulieren und in einer Behandlungsphase zu halten und wobei die Vorrichtung mindestens einen Kontaktsensor umfasst.

## Beschreibung

Die Erfindung betrifft eine Vorrichtung zur Behandlung von Juckreiz und/oder Herpes auf einer Haut, wobei eine Regelvorrichtung konfiguriert ist, eine Behandlungsfläche auf einer der Haut zugewandten Außenseite durch Erhitzen mindestens eines Heizelementes in einer Aufheizphase auf eine Kontakttemperatur während eines Kontaktes der Behandlungsfläche mit der Haut zu regulieren und in einer Behandlungsphase zu halten und wobei die Vorrichtung mindestens einen Kontaktsensor umfasst.

### Hintergrund und Stand der Technik

Juckreiz (Pruritus) ist eine subjektiv unangenehme, auf die Haut oder Schleimhaut bezogene Sinneswahrnehmung. Sie kann lokal begrenzt sein oder aber den ganzen Körper betreffen. Häufig geht Juckreiz mit einem brennenden, stechenden oder kribbelnden Gefühl einher, welches die betroffene Person oftmals durch Kratzen, Scheuern, Rubbeln, Drücken, Kneten oder Reiben versucht zu lindern. Daher kommt es bei Juckreiz gehäuft zu weiteren pathologischen Erscheinung der Haut wie Kratzspuren, offene Wunden, Krustenbildung und Hautinfektionen. Die Fachwelt geht davon aus, dass Juckreiz über Schmerzrezeptoren der Haut vermittelt wird und über das vegetative Nervensystem zum Gehirn geleitet wird. Die Ursachen von Juckreiz können sehr vielfältig sein. Neben trockener Haut, fehlender Feuchtigkeitszufuhr oder Allergien kann Juckreiz auch durch äußere Einwirkungen und Hautreizungen, wie z.B. durch Stiche von Mücken oder nach Kontakt mit Nesseltieren entstehen. Juckreiz kann eine Reaktion auf chemische, mechanische oder thermische Reize sein. Aus medizinischer Sicht spannen die Ursachen oder Grunderkrankungen, welche zu Juckreiz führen ein breites Spektrum dermatologischer und internistischer Erkrankungen auf.

Zur medikamentösen Behandlung von den Symptomen des Juckreizes sind eine Reihe von Medikamenten oder kosmetischen Produkten bekannt. So werden ätherische Öle insbesondere umfassend Menthol, Thymol oder Campher verwandt, um kurzfristig eine Kühlung zu erzeugen. Auch Hautpflegemittel wie beispielsweise Cremen oder Lotion können durch eine Erhöhung des Feuchtigkeitsgehaltes der Haut eine schmerzlindernde Wirkung entfalten. Darüber hinaus stellen Antihistaminika hilfreiche Therapiemöglichkeiten dar.

Aus dem Stand der Technik ist es jedoch auch bekannt durch Einbringung einer Wärmemenge in den Einstich von Insekten das Auslösen eines Juckreizes zu mindern. Eine Vorrichtung für eine lokale, thermische Behandlung insbesondere von Mückenstichen wird in der EP 1231875 B1 beschrieben.

Die Anwendungsmöglichkeiten einer hyperthermischen Behandlung erstrecken sich weiterhin auf Herpeserkrankungen. Aus der DE 102005002946 A1 ist eine Vorrichtung zur Behandlung von Herpeserkrankungen bekannt. Die Hitzeapplikation führt zum einen zu einer Eindämmung der Vermehrung der ursächlichen Krankheitserreger durch eine neutralisierende Wirkung auf die Herpes-simplex-Viren. Zum anderen kommt es durch die kurzzeitige Wärmebehandlung zu einer Überlagerung des Juckreizes der Herpeserkrankung durch die Stimulation temperatursensitiver Nerven.

Die aus dem Stand der Technik bekannten Vorrichtungen zur hyperthermischen Behandlung zeichnen sich durch vielfältige Einsatzmöglichkeiten zur Linderung der Symptome von Insektenstichen, Herpeserkrankungen, Quallenstichen oder anderen mit Juckreiz einhergehenden Erkrankungen aus. Die Vorrichtungen weisen jedoch auch Nachteile auf.

Bei der Erhitzung der typischerweise genutzten Heizplatten in den bekannten Vorrichtungen kann es zu subjektiv empfundenen Temperaturschwankungen und einer starken Schmerzwahrnehmung aufgrund zu hoher Temperaturen kommen. Dies liegt das an einer unzureichend bekannten und daher zu unpräzise geregelten Kontakttemperatur der bekannten Vorrichtungen. Dies kann dazu führen, dass die Probanden die Behandlung abbrechen und sich der Therapieerfolg mindert.

### Aufgabe der Erfindung

Aufgabe der Erfindung war es eine Vorrichtung bereitzustellen, welche die Nachteile des Standes der Technik beseitigt. Insbesondere sollte eine Vorrichtung bereitgestellt werden, welche sich zur Behandlung von Juckreiz eignet und sich durch eine erhöhte Effektivität, Anwendungskomfort sowie damit verbundener Compliance auszeichnet.

### Zusammenfassung der Erfindung

In einer bevorzugten Ausführungsform betrifft die Erfindung somit eine Vorrichtung zur Behandlung von Juckreiz und/oder Herpes auf einer Haut, umfassend
a) mindestens eine Behandlungsfläche und
b) eine Regelvorrichtung zur Regulation der Temperatur der Behandlungsfläche,
dadurch gekennzeichnet, dass die Regelvorrichtung konfiguriert ist die Behandlungsfläche auf einer der Haut zugewandten Außenseite durch Erhitzen mindestens eines Heizelementes in einer Aufheizphase auf eine Kontakttemperatur während eines Kontaktes der Behandlungsfläche mit der Haut zu regulieren und in einer Behandlungsphase zu halten und wobei die Vorrichtung mindestens einen Kontaktsensor umfasst.

Um eine hyperthermische Behandlung durchzuführen sorgt die erfindungsgemäße Regulationsvorrichtung dafür, dass die Behandlungsfläche durch Erhitzen mindestens eines Heizelementes auf eine Kontakttemperatur, z. B. von 43 - 47 °C, während eines Kontaktes der Behandlungsfläche mit der Haut geführt wird. Die Kontakttemperatur bezeichnet im Sinne der Erfindung die Temperatur, welche die Behandlungsfläche an einer der Haut zugewandten Außenseite aufweist, während diese im Kontakt der Hautstelle vorliegt. Erfindungsgemäß wird mithin zwischen einer Kontakttemperatur und einer Nichtkontakt-Temperatur der Behandlungsfläche unterschieden. Hierbei meint die Nichtkontakt-Temperatur die Temperatur der Behandlungsfläche, wenn diese nicht die Haut kontaktiert, sondern beispielsweise ohne thermische Last lediglich Luft kontaktiert.

Bei bekannten hyperthermischen Vorrichtungen erfolgt i.d.R. eine Regulation der Temperatur der Behandlungsfläche durch ein Heizelement auf Basis einer Nichtkontakt-Temperatur. D.h. die Parameter der Regulationsvorrichtungen werden anhand standardisierter Messungen eines Temperaturverlaufs ohne thermische Last durchgeführt. Bei einer derartigen Regulation wird auch nicht zwangsläufig unterschieden zwischen einer Regulation der Temperatur der Behandlungsfläche an der Seite, welcher der Haut zu- oder abgewandt ist.

Von den Erfindern wurde erkannt, dass insbesondere bei der Behandlung sensitiven Stellen, eine derartige Regulation nicht ausreichend präzise ist. So kann es zu Schwankungen und subjektiv stark wahrgenommen Schmerzen kommen, welche zu einer verminderten Compliance der Probanden (Bereitschaft der Probanden zur aktiven Mitwirkung) und somit zu einem verminderten Therapieerfolg führen.

Insbesondere bei der Behandlung von z. B. Herpes an sensitiven Stellen wie den Lippen, wofür die Vorrichtung ebenfalls bevorzugt geeignet ist, ist eine derartige Regulation nicht ausreichend präzise ist. Aufgrund der dünneren Haut kommt es z. B. an den Lippen leichter zu physiologischen Schäden. Auch ist die Schmerzempfindlichkeit aufgrund dessen höher.

Als Außenseite der Behandlungsfläche, wird im Sinne der Erfindung jene Seite der Behandlungsfläche bezeichnet, welche bei der Vorrichtung von außen zugänglich ist und somit der Seite entspricht, welche bei einer Anwendung der Vorrichtung der Haut zugewandt ist. Wenn der Begriff "einer der Haut zugewandten Außenseite der Behandlungsfläche" verwandt wird, hat dies vorwiegend erläuternden Charakter. Für eine hyperthermische Vorrichtung wird stets eine Außenseite und Innenseite der Behandlungsfläche definiert sein. Die Innenseite bezeichnet dabei bevorzugt die von der Haut abgewandte Seite. Im Falle eines Gehäuses wird es bevorzugt sein, dass die Behandlungsfläche in der Gehäusefläche integriert vorliegt, wobei die Innenseite im Gegensatz zur Außenseite nicht von außen zugänglich oder sichtbar ist.

Zur Einstellung der präzisen Kontakttemperatur kann die Vorrichtung Temperatursensoren aufweisen, welche die Temperatur der Behandlungsfläche während eines Kontaktes mit der Haut messen und die Heizelemente demensprechend regulieren. In Fällen, in denen ein Temperatursensor nicht unmittelbar an der der Haut zugewandten Außenseite die Temperatur der Behandlungsfläche misst, beispielsweise, weil dieser auf der Innenseite einer Behandlungsfläche installiert ist, kann die Zieltemperatur der Regelvorrichtung für den Messwert des Temperatursensors so angepasst werden, dass die Kontakttemperatur präzise im vorgegebenen Bereich gehalten wird. Beispielsweise kann anhand von experimentellen Versuchen oder Berechnungen des Wärmeflusses eine entsprechend höhere Zieltemperatur eingestellt werden. So wird gewährleistet, dass beim Kontakt der Behandlungsfläche mit der Haut die Kontakttemperatur vorliegt. Vorteilhafterweise konnten Versuche zeigen, dass die Haut eines Menschen über verschiedene Probanden hinweg ähnliche thermische Eigenschaften aufweisen, sodass die experimentellen oder theoretischen Ergebnisse zuverlässig übertragbar sind. Es hat sich ebenso insbesondere gezeigt, dass sich die thermischen Eigenschaften gleicher Extremitäten eines Probanden oftmals stark ähneln, so dass diese thermischen Eigenschaften zur Bestimmung der Kontakttemperatur herangezogen werden können. Eine Vorrichtung kann z. B. für die Behandlung bestimmter Extremitäten wie bspw. der Lippen geeignet sein und deren thermische Eigenschaften berücksichtigen. Auch können die zu behandelnden Extremitäten bevorzugt eingestellt oder automatisch erkannt werden, so dass sie bei einer Behandlung berücksichtigt werden.

Während des Zyklus einer hyperthermischen Behandlung wird die der Haut zugewandte Außenseite der Behandlungsfläche zunächst in einer Aufheizphase auf die Kontakttemperatur geführt. Es ist bevorzugt, dass die Aufheizphase keinen längeren Zeitraum bedarf. Bevorzugt sollte die Aufheizphase nicht mehr als 10 s, besonders bevorzugt nicht mehr als 3 s betragen. Ausgezeichnete Ergebnisse konnten bei Aufheizzeiten von 1-3 Sekunden erzielt werden. Im Anschluss an die Aufheizphase wird die Kontakttemperatur für die Dauer der Behandlungsphase gehalten. Es kann bevorzugt sein, dass die Kontakttemperatur während der Behandlungsphase konstant ist. Es kann aber auch bevorzugt sein, dass die Kontakttemperatur während der Behandlungsphase innerhalb der vorgegebenen Grenzen variiert.

Im Sinne der Erfindung meint die Behandlungsphase bevorzugt einen zusammenhängenden Zeitraum während dessen die Regulationsvorrichtung die der Haut zugewandten Seite der Behandlungsfläche eine Kontakttemperatur in einem vorgegebenen Bereich, z. B. von 43-47°C, hält. Vor der Behandlungsphase, d.h. während der Aufheizphase, liegt die Kontakttemperatur unterhalb der Werte. Nach Beendigung der Behandlungsphase erfolgt bevorzugt kein Erhitzen des mindestens eines Heizelementes durch die Regelungsvorrichtung mehr, sodass die Kontakttemperatur unter den vorgegebenen Bereich fällt. Eine Behandlungsphase kann z. B. 1 bis 10 Sekunden betragen.

Durch die Regulation der Außenseite der Behandlungsfläche auf eine vorgegebene Kontakttemperatur wird eine definierte Wärmemenge auf die Hautstelle auf kontrollierte Weise gebracht. Der definierte Wärmepuls führt zu einer überraschend effektiven Therapie von Juckreiz und Herpeserkrankungen, insbesondere von Lippenherpes, ohne dass unangenehme Schmerzen oder gar Verbrennungen auftreten.

Studien haben gezeigt, dass das Risiko einer Verbrennung bei einem Temperaturniveau von bspw. 44°C bis 51° mit jedem Grad Celsius um einen Faktor zwei zunimmt. Auch berichten Probanden, dass ab einer Temperatur von 47,5°C bis 48,5°C Wärme an einer Haut in Form von stechendem Schmerz wahrgenommen wird. Unter 47°C erscheint die Temperaturbevorzugt sehr viel erträglicher.

Demgegenüber wurde z. B. erkannt, dass eine Thermolabilität des DNA-bindenden Proteins ICP8 ausgenutzt werden kann, um die Replikation der Herpesvirus DNA wirksam zu unterbinden. Studien belegen eine Reduktion der Bindungsaktivität des Proteins an der Virus-DNA um ca. 50% bei einer Temperatur von 45°C. Insbesondere wurde erkannt, dass eine besonders starke Überlagerung des Juckempfindens erreicht werden kann, wenn in den betreffenden Hautpartien lokal der Thermo- und Capsaicinrezeptor TRPV1 aktiviert wird. Der TRPV1 ist bei akutem Hitze-induzierten Schmerz in gesunder Haut beteiligt und reguliert beispielsweise das Heißempfinden bei Temperaturen um 45° bis 50 °C. Eine Aktivierung von TRPV 1 unterdrückt zusätzlich Spannungsgefühle und Juckreiz und damit die Begleitsymptomatik der Herpeserkrankung. Daher sind präzise geregelte Kontakttemperaturen bei der Behandlung von Herpes von Bedeutung. Auch zur Neutralisation von Insektengiften haben sich präzise geregelte Kontakttemperaturen etwa in diesem Bereich als vorteilhaft erwiesen.

Einerseits sollte somit z. B. gegen Herpes oder auch Juckreiz eine möglichst hohe Temperatur für das Einbringen der Wärme gewählt werden, anderseits kann ein damit verbundener Schmerz dazu führen, dass die Behandlung vorzeitig abgebrochen wird und sich somit kein Erfolg einstellt.

Deswegen können beispielsweise vorstehend genannte Temperaturen, z. B. zur Behandlung von Herpes, als Kontakttemperatur bevorzugt sein.

Im Sinne der Erfindung bezeichnet die Behandlungsfläche bevorzugt eine Materialfläche der Vorrichtung, welche während der Behandlung in direktem thermischen Kontakt mit der Hautpartie steht.

Die Behandlungsfläche kann eine zusammenhängende Fläche darstellen. Es kann auch bevorzugt sein, dass die Behandlungsfläche aus mehreren nicht zusammenhängenden Teilflächen besteht. Die Größe der Behandlungsfläche bezieht sich bevorzugt jeweils auf die gesamte Kontaktfläche, über welche eine Hautpartie einen Wärmeimpuls erfährt. Im Falle einer Behandlungsfläche, welche aus mehreren Teilflächen besteht, entspricht die Größe der Behandlungsfläche bevorzugt der Summe der einzelnen Teilflächen. Eine solche Aufteilung in Teilflächen kann bei bestimmten Erscheinungsformen von Herpes vorteilhaft sein, ebenso wie bei der Behandlung bestimmter Körperstellen.

Es ist bevorzugt, dass die Behandlungsfläche mit Hilfe von mindestens einem Heizelement auf die gewünschte Kontakttemperatur gebracht wird. In einer bevorzugten Ausführungsform entspricht die Behandlungsfläche der Oberfläche einer Heizplatte, welche mit Hilfe eines Heizelementes erhitzt wird, wobei zum Beispiel ein Halbleiter-Bauelement zum Einsatz kommen kann. Die Behandlungsfläche kann jedoch auch eine homogene Materialfläche bezeichnen, welche durch mehrere Heizelemente temperiert wird. Beispielsweise kann es bevorzugt sein zwei oder vier Heizelemente zu verwenden, um die Behandlungsfläche besonders homogen und schnell auf die Kontakttemperatur zu führen. Es kann auch bevorzugt sein eine Heizplatte umfassend ein Heizelement zu beschichten. In dem Fall wird unter der Behandlungsfläche bevorzugt die Beschichtung der Heizplatte verstanden, so dass die Kontakttemperatur bevorzugt stets jene Temperatur angibt, welche an der der Haut zugewandten Seite der Behandlungsfläche, während eines Kontaktes der Behandlungsfläche mit der Haut vorliegt.

Im Sinne der Erfindung ist die Regelvorrichtung bevorzugt ein Prozessor, ein Prozessorchip, Mikroprozessor oder ein Mikrokontroller, welcher konfiguriert ist, um die Temperatur der Behandlungsfläche mit Hilfe des mindestens einen Heizelementes gemäß der vorgegebenen Werte für die Kontakttemperatur zu regulieren.

Bevorzugt wird unter dem Heizelement jenes Bauelement bezeichnet, welches durch die Regelvorrichtung u.a. durch Anlegen eines elektrischen Stromes erhitzt werden kann. Das mindestens eine Heizelement ist ein Bauelement, für welches verschiedene Ausführungsformen aus dem Stand der Technik hinreichend bekannt sind. So kann das Heizelement einen Leistungswiderstand umfassen, bei welchem in Abhängigkeit des Stromflusses eine wohldefinierte Temperatur generiert wird. Bevorzugt kann zur quantitativen Steuerung des Stromflusses durch das Heizelement ein Feldeffekttransistor (FET) verwandt werden. Es kann aber auch bevorzugt sein, einen FET selbst als Heizelement einzusetzen. Hierbei wird Energiedissipation im Transistor selbst genutzt, um Wärme zu generieren und die Behandlungsfläche auf die Kontakttemperatur zu bringen. FETs sind als Heizelemente besonders bevorzugt, da diese aufgrund ihrer geringen Größe eine kleine Dimensionierung erlauben. Weiterhin sind FETs besonders reaktiv und gewährleisten durch eine sehr dynamische Wärmeerzeugung und Wärmeabgabe ein besonders schnelles Ansprechverhalten der Heizelemente.

Bevorzugt kann die Regelvorrichtung durch die Vorgabe der Stromzufuhr zum Heizelement kontrollieren, welche Kontakttemperatur vorliegt. Beispielsweise kann mit Hilfe einer Kalibration die Korrelation zwischen Stromfluss und/oder Spannung an dem Heizelement mit der Kontakttemperatur während des Kontaktes der Behandlungsfläche mit der Haut ermittelt werden, sodass auf Basis der Kalibrierung stets eine gewünschte Kontakttemperatur eingestellt werden kann.

Es kann aber auch bevorzugt sein, die Kontakttemperatur durch die Regelvorrichtung mit Hilfe einer Feedbackschleife zu regulieren. So kann es bevorzugt sein einen Temperatursensor einzusetzen, welcher an einer Position der Behandlungsfläche eine Temperatur misst, wobei die Regelvorrichtung anhand der Temperaturdaten die Stromzufuhr zum Heizelement reguliert. Zu diesem Zweck kann die Regelvorrichtung beispielsweise einen Mikroprozessor umfassen.

Im Sinne der Erfindung wird unter einem Mikroprozessor bevorzugt eine Datenverarbeitungsvorrichtung, d.h. ein Prozessor, verstanden, welcher sich durch kleine Abmaße im Bereich von einigen mm auszeichnet und wobei bevorzugt alle Bausteine des Prozessors auf einem Mikrochip oder auch integriertem Schaltkreis (engl. *integrated circuit, IC*). Der Mikroprozessor kann bevorzugt auch ein Mikrokontroller sein, welcher neben dem Prozessor weitere periphere Elemente auf dem Mikrochip integriert und beispielsweise auch über einen Datenspeicher verfügt.

Es ist weiterhin bevorzugt, dass der Mikroprozessor auf einer Leiterplatte (engl. *printed circuit board,* PCB) installiert vorliegt. Außer dem Mikroprozessor liegen auf dem PCB weiterhin bevorzugt die Heizelemente sowie die Temperatorsensoren installiert vor. Diese bevorzugte Ausführungsform erlaubt eine äußert kompakte und ebenso robuste Bauweise der Vorrichtung und eine besonders intelligente Temperaturregulation mit Hilfe des Mikroprozessors. So ist der Mikroprozessor nicht nur in der Lage die gemessenen Temperaturdaten auszuwerten und in einer Steuerung der Heizelemente zu übersetzen, sondern kann weiterhin andere Parameter wie Fehlermeldungen und Benutzerinput schnell und zuverlässig berücksichtigen.

In einer bevorzugten Ausführungsform der Erfindung ist die Vorrichtung dadurch gekennzeichnet, dass der Mikroprozessor und das Heizelement und der optionale, eine Temperatursensor auf einem *printed circuit board* (PCB) installiert sind, wobei mindestens das Heizelement und der Temperatursensor mit Hilfe eines Schutzlackes beschichtet sind. Im Sinne der Erfindung wird unter dem Schutzlack bevorzugt ein Lack oder eine Farbe verstanden, welcher Bauteile des PCBs vor Umwelteinflüssen schützen soll.

Der Schutzlack wirkt zu diesem Zweck bevorzugt elektrisch isolierend und ist wasserbeständig. Die Eigenschaft der elektrischen Isolation kann bevorzugt anhand des Oberflächenwiderstandes oder engl. *surface insulation resistance* (SIR) quantifiziert werden. Der SIR kann bevorzugt beispielsweise durch Leckströme zwischen den Bauteilen der Leiterplatte gemessen werden. Ein hoher Widerstand entspricht einer guten elektrischen Isolation. Wasserbeständig meint bevorzugt, dass auch bei einer hohen Luftfeuchtigkeit oder dem Eindringen von Wasser die lackierten elektronischen Bauteile intakt bleiben und es zu keinem Kurz-schluss kommt. Die Wasserbeständigkeit kann beispielsweise ebenfalls unter Messung der SIR unter Bedingungen mit hoher Luftfeuchtigkeit getestet werden.

Im Stand der Technik ist eine Vielzahl von Schutzlacken bekannt, welche bevorzugt verwandt werden können. Beispielhaft seien Schutzlacke auf Acryl-, Silikon- oder Polyurethanbasis genannt. Durch die Applikation des Schutzlackes im Bereich der Heizelemente und Temperatursensoren werden diese vor Ablagerungen wirksam geschützt, sodass Fehlmessungen der Temperatursensoren vermieden werden können. Dies erhöht zum einen die Genauigkeit mit welcher die Kontakttemperatur eingestellt werden kann und vermeidet zum anderen, dass aufgrund einer Fehlmessung der Temperatur es zu einem Überhitzen der Behandlungsfläche kommt.

Des Weiteren umfasst die Vorrichtung mindestens einen Kontaktsensor. Im Sinne der Erfindung bezeichnet der Kontaktsensor eine Einheit, welche anhand von Messdaten und deren Analyse eine Aussage darüber treffen kann, ob die Behandlungsfläche in Kontakt mit einer Haut, bspw. einer Lippe, ist oder nicht. Vorzugsweise umfasst der Kontaktsensor hierzu einen Sensor oder eine Messeinheit, welche mit der Regelungsvorrichtung verbunden, wobei die Regelungsvorrichtung die Auswertung der Messdaten vornehmen kann.

Mittels des Kontaktsensors und der Information darüber zu welchem Zeitpunkt die Behandlungsfläche die Haut kontaktiert, kann eine besonders präzise Regulation des Wärmeflusses zur Behandlung von Herpes erzielt werden. Beispielsweise kann der Beginn der Aufheizphase davon abhängig gemacht werden, ob ein Kontakt mit der Haut vorliegt. Auch der Zeitraum der Behandlungsphase kann zuverlässig aufgezeichnet werden, um erfolgte Behandlungen zu überwachen und ggf. weitere Anwendungen daraufhin anzupassen. Auch hinsichtlich von Sicherheitsaspekten erlaubt der Kontaktsensor eine verbesserte Kontrolle. So kann mit Hilfe eines Kontaktsensors vermieden werden, dass sich die Behandlungsfläche ohne Wissen oder Wollen des Nutzers aufheizt.

In einer bevorzugten Ausführungsform der Erfindung umfasst die Vorrichtung mindestens einen Temperatursensor zur Messung der Temperatur der Behandlungsfläche, wobei die Regelvorrichtung basierend auf den Messdaten des Temperatursensors das mindestens eine Heizelement reguliert.

Im Sinne der Erfindung ist ein Temperatursensor bevorzugt ein elektrisches oder elektronisches Bauelement, welches in Abhängigkeit der Temperatur am Sensor ein elektrisches Signal generiert. Im Stand der Technik sind eine Vielzahl von Temperatursensoren bekannt wie beispielsweise Halbleiter-Temperatursensoren, Widerstandstemperatursensoren, pyroelektrische Materialien, Thermoelemente oder Schwingquarze.

Die Regelvorrichtung ist weiterhin bevorzugt derart konfiguriert, dass diese die Messwerte der Temperatursensoren aufnehmen und auswerten kann, um eine Regulation des oder der Heizelemente zu bewirken. Die Regulation der Heizelemente kann bevorzugt mithilfe des Anliegens eines elektrischen Stroms oder einer Spannung erfolgen. Es ist besonders bevorzugt, dass der Temperatursensor die Temperatur der Behandlungsfläche direkt misst, d.h. dass der Temperatursensor in Kontakt mit der Behandlungsfläche ist, wobei sich der Temperatursensor sowohl auf der Innenseite der Behandlungsfläche, als auch auf der Außenseite der Behandlungsfläche befinden kann oder aber in die Behandlungsfläche implementiert ist.

Die Regelvorrichtung ist so konfiguriert, dass diese auf Basis der gemessenen Temperatur des Temperatursensors zuverlässig die Kontakttemperatur einstellt. Wird beispielsweise der Temperatursensor auf der Innenseite der Behandlungsfläche angebracht, so wird die Regelvorrichtung dazu konfiguriert sein, als Zieltemperatur an der Innenseite der Behandlungsfläche eine höhere Temperatur einzustellen, als dies an der maßgeblichen Außenseite der Behandlungsfläche während des Kontaktes gewünscht ist. Die Differenz zwischen einer solchen Zieltemperatur und der zu erreichenden Kontakttemperatur kann der Regelvorrichtung anhand theoretischer Vorhersagen über den Wärmefluss innerhalb der Behandlungsfläche beim Anlegen an einer Haut oder aber auf Basis von Kalibrationsmessungen als Referenzdaten bereitgestellt werden.

Es kann aber auch bevorzugt sein, dass der Temperatursensor nicht die Behandlungsfläche direkt, sondern die Heizelemente oder einen Materialpunkt zwischen den Heizelementen und der Behandlungsfläche kontaktiert und überwacht. Im Falle von mehreren Heizelementen, welche die Behandlungsfläche erhitzen, kann es beispielsweise auch bevorzugt sein, den Temperatursensor zwischen den Heizelementen zu platzieren. Aus den Messdaten für die Temperatur über die Heizelemente oder einem Messpunkt in einer bestimmten Distanz zur Behandlungsfläche kann ebenfalls auf die Temperatur der Behandlungsfläche geschlossen werden.

Die Kontakttemperatur meint bevorzugt die durchschnittliche Temperatur der Außenseite der Behandlungsfläche, während diese während der Anwendung des Gerätes eine Haut kontaktiert.

Eine Auswertung der Temperatur der Behandlungsfläche erlaubt eine besonders präzise Regulation des mindestens einen Heizelementes, um eine optimale Temperaturverteilung auf der Außenseite der Behandlungsfläche und somit Wärmeübertragung an die zu behandelnden Hautpartien sicherzustellen. Insbesondere im Hinblick auf die Anwendung der Vorrichtung für sensible Hautpartien zeichnet sich Ausführungsform durch eine gezieltere und kontrollierte Regelung aus.

In dieser Ausführungsform der Erfindung ist die Vorrichtung dadurch gekennzeichnet, dass die Regelvorrichtung auf Basis einer Korrelation der Messdaten des Temperatursensors und Daten über die Ansteuerung des Heizelementes feststellen kann, ob die Behandlungsfläche in Kontakt mit der Haut vorliegt. In dieser Ausführungsform wird der Kontaktsensor durch einen Temperatursensor und einer Regelvorrichtung zur Steuerung der Heizelemente gebildet. Grundlage der Kontaktmessung ist die Erkenntnis, dass der notwendige Stromfluss zum Erreichen oder Aufrechterhalten einer Temperatur davon abhängt, ob die Behandlungsfläche eine thermische Last (z.B. eine Haut) kontaktiert. Im Falle eines Aufheizens der Behandlungsfläche beim Hautkontakt erfolgt ein Wärmeübertrag, welcher durch eine erhöhte Energiezufuhr an die Heizelemente ausgeglichen werden muss. Durch Auswertung des Strom- und Temperaturverlaufes können zuverlässige Aussagen darüber getroffen werden, ob die Behandlungsfläche eine Haut kontaktiert. Bevorzugt kann der Regelungsvorrichtung zu diesem Zweck Referenzdaten bereitgestellt werden.

In einer bevorzugten Ausführungsform der Erfindung umfasst die Regelvorrichtung Referenzdaten über eine Korrelation der Temperatur der Behandlungsfläche mit der Ansteuerung des mindestens einen Heizelementes im Falle, dass die Behandlungsfläche in Kontakt mit der Haut oder mit Luft vorliegt. Die Referenzdaten können beispielsweise Verhältnisse aus gemessener Temperatur und dafür notwendiger Stromzufuhr umfassen. Besonders bevorzugt umfassen die Referenzdaten derartige Verhältnisse für einen Temperaturverlauf, sodass anhand der Messung des aktuellen Verhältnisses aus Temperatur und Stromzufuhr besonders präzise bestimmt werden kann, ob die Behandlungsfläche eine Haut kontaktiert. Vorteilhafterweise kann mithilfe einer derartigen Regulation nicht nur ein Kontakt mit der Haut im Vergleich zur Luft, sondern ebenfalls ein Kontakt mit der Haut im Vergleich zu Materialien mit anderen thermischen Eigenschaften zuverlässig unterschieden werden.

In einer weiteren bevorzugten Ausführungsform können die Referenzdaten die durchschnittlich an der Haut oder der Luft abgegebene Wärmemenge umfassen. Dabei können die Referenzdaten Korrelationen zwischen Kontakttemperatur und abgegebener Wärme enthalten.

In einer weiteren bevorzugten Ausführungsform der Erfindung ist die Vorrichtung dadurch gekennzeichnet, dass die Behandlungsfläche und aus einem Material besteht, welches eine Wärmeleitfähigkeit bei 45°C zwischen 20 W/mK und 400 W/mK, bevorzugt zwischen 100 und 350 W/mK aufweist. Die Wärmeleitfähigkeit (auch als Wärmeleitzahl bezeichnet) charakterisiert bevorzugt die thermischen Eigenschaften des Materials, aus welchem die Behandlungsfläche gefertigt ist. Die Wärmeleitfähigkeit gibt an, wie hoch die Wärmemenge ist, welche durch die Behandlungsfläche geleitet wird, wenn an dieser ein Temperaturgradient anliegt.

Neben der Wärmeleitfähigkeit hängt der Wärmetransport von der Dicke der Behandlungsfläche, der Größe der Behandlungsfläche und dem Temperaturunterschied zwischen der Innenseite der Behandlungsfläche (Kontakt mit den Heizelementen) und der äußeren Seite der Behandlungsfläche (Kontakt mit der Haut) ab. Die Wärmeleitfähigkeit wird bevorzugt als Verhältnis der transportierten Wärmeleistung Watt (W) pro Temperaturdifferenz in Kelvin (K) und pro Meter (m) angegeben. Da die Wärmeleitfähigkeit sich weiterhin in Abhängigkeit der Temperatur leicht ändern kann, wird vorliegend die Referenztemperatur mit 45 °C angegeben.

Die Dicke der Behandlungsfläche bezeichnet weiterhin bevorzugt die Ausdehnung der Behandlungsfläche zwischen der äußersten Fläche, welche die Haut kontaktiert und der innersten Fläche, an welcher die Heizelemente anliegen. In einigen Ausführungsformen kann die Dicke der Behandlungsfläche zwischen 0,2 mm und 5 mm, bevorzugt zwischen 0,5 mm und 2 mm aufweisen.

In einer bevorzugten Ausführungsform umfasst die Behandlungsfläche Keramik oder Gold. Es ist besonders bevorzugt, dass die Behandlungsfläche aus Gold oder Keramik besteht. Die Materialien Keramik und Gold fallen zum einen in experimentell ermittelte, bevorzugte Bereiche der Wärmeleitfähigkeit.

Darüber hinaus zeichnen sich sowohl Keramik als auch Gold auf überraschende Weise zur Behandlung von Herpes aus. Insbesondere entfalten die Materialien bei den Patienten eine gesteigert empfundene Schmerzüberlagerung. Dies ist insofern überraschend, als dass der Effekt über den reinen Temperatureffekt durch thermisch vergleichbare Materialien hinausgehen kann.

Darüber hinaus zeichnen sich Gold und insbesondere Keramik durch eine überraschende hohe biologische Verträglichkeit aus, welche gepaart mit einer besonders geringen Ausprägung von Allergien gegenüber diesen Materialien die Anwendung in einem Gerät zur Behandlung von vorwiegend dermatologischen Erkrankungen besonders auszeichnet.

Eine besonders bevorzugte Keramik ist Aluminiumnitrid. Diese zeichnet sich in besonders starkem Maße durch eine außergewöhnliche biologische Verträglichkeit und ausgezeichnete thermische Eigenschaften aus. Zudem ist eine Behandlungsfläche aus Aluminiumnitrid besonders stark elektrisch isolierend, sodass eine erhöhte Sicherheit in der Anwendung gewährleistet werden kann. Dies führt insbesondere in Verbindung mit der Verwendung eines Schutzlackes besonders vorteilhaft und führt zu einer synergistischen Erhöhung der Sicherheit.

Dabei macht sich der Vorteil der Verwendung von Keramik und insbesondere Aluminiumnitrid bemerkbar, dass die Behandlungsfläche problemlos und ohne Verschlechterung der Oberfläche mit einem Desinfektionsmittel desinfiziert werden kann, wodurch eine antimikrobakterielle Wirkung mit den oben genannten Vorteilen erzielt wird. Durch die erhöhte Sicherheit bei einer Verwendung von Schutzlack in Verbindung mit einer Behandlungsfläche aus Keramik und insbesondere Aluminiumnitrit können problemlos und sicher flüssige Desinfektionsmittel verwendet werden.

Es können jedoch auch bei einer Fläche aus Gold Desinfektionsmittel verwendet werden.

Die Verwendung von Desinfektionsmitteln zur Desinfektion einer Behandlungsfläche oder gar des ganzen Gerätes macht insbesondere bei einer Vorrichtung zur Behandlung von Herpeserkrankungen großen Sinn, da Herpes bekanntermaßen extrem ansteckend ist und eine Vorrichtung nur durch eine gründliche Desinfektion von mehr als einer Person verwendet werden kann.

In einer bevorzugten Ausführungsform ist die Vorrichtung dadurch gekennzeichnet, dass der Temperatursensor an der Innenseite der Behandlungsfläche vorliegt und die Behandlungsfläche durch eine Keramikschicht mit einer Schichtdicke zwischen 50 µm und 2000 µm, bevorzugt zwischen 50 µm und 1500 µm und besonders bevorzugt 50 µm und 1000 µm gebildet wird.

In einer bevorzugten Ausführungsform ist die Vorrichtung dadurch gekennzeichnet, dass der Temperatursensor an der Innenseite oder -fläche der Behandlungsfläche vorliegt und die Behandlungsfläche durch eine Goldschicht mit einer Schichtdicke zwischen 5 µm und 2000 µm, bevorzugt zwischen 50 µm und 1500 µm und insbesondere zwischen 50 µm und 1000 gebildet wird.

Durch die vorgenannten bevorzugten Schichtdicken ist es möglich besonders präzise die Kontakttemperatur während der Anwendung der Vorrichtung einzustellen und in einem engen Temperaturbereich zu halten. Zwar ist es möglich auch bei dickeren Behandlungsflächen ausgehend von einer Temperaturmessung auf der Innenseite der Behandlungsfläche Rückschlüsse über die Kontakttemperatur auf Basis von Kalibrationskurven oder theoretischen Berechnungen zu ziehen. Die genannten Schichtdicken von bevorzugt weniger als 2000 µm, weniger als 1500 µm und weniger als 1000 µm minimieren jedoch Fehlerquellen aufgrund von Toleranzen in der Herstellung oder Veränderungen an der Vorrichtung (Feuchtigkeiten, Abnutzungen etc.) die zu veränderten Wärmeflüssen in der Behandlungsfläche führen können.

Durch die Implementation der Temperatursensoren auf der Innenseite maximal 200 µm unterhalb der Außenseite der Behandlungsfläche kann eine deutlich zuverlässige Messaussage über die Kontakttemperatur getroffen werden. Im Rahmen einer Feedback-Regulation wird die Zieltemperatur für den Temperatursensor an der Innenseite sich nur geringfügig von der anvisierten Kontakttemperatur abweichen, wodurch Störfaktoren weitestgehend eliminiert werden können.

In einer weiteren bevorzugten Ausführungsform der Erfindung ist die Vorrichtung dadurch gekennzeichnet, dass die Behandlungsfläche durch eine Keramik gebildet wird und der Temperatursensor in die Behandlungsfläche integriert vorliegt. In dieser Ausführungsform kann vorteilhafterweise unabhängig von der Dicke der Schicht der Behandlungsfläche der Temperatursensor äußerst nahe an die der Haut zugewandten Außenseite gebracht werden.

Die Ausführungsform zeichnet sich mithin durch einen zusätzlichen konstruktiven Freiheitsgrad hinsichtlich der Schichtdicke aus bei gleichermaßen präziser Regulation der Kontakttemperatur. Beispielsweise kann es bevorzugt sein eine Behandlungsfläche aus Keramik mit einer Schichtdicke von 0,5 bis 2 mm zu verwenden, wobei der Temperatursensor als Dünnschichtsensor innerhalb der Behandlungsfläche integriert vorliegt. Im Gegensatz zu bekannten Messmethoden bei der hyperthermischen Behandlung kann hierdurch ein besonders präzises Abbild des Wärmeflusses, während der Behandlung gewonnen werden.

In einer weiteren bevorzugten Ausführungsform umfasst der Kontaktsensor einen optischen Detektor, einen kapazitiven Sensor, einen taktilen Sensor und/oder ein Pyrometer. Beispielsweise kann der Kontaktsensor einen optischen Sensor umfassen, welcher die Messdaten über die Lichtverhältnisse an die Regelungseinheit übermittelt. Sofern der optische Sensor nahe oder in der Behandlungsfläche installiert vorliegt, weist ein Abfallen an Helligkeit darauf, dass die Behandlungsfläche eine Haut kontaktiert. Es kann aber auch bevorzugt sein, dass ein Kontaktsensor eine Einheit aus einem Temperatursensor und der Regelvorrichtung zur Regulation der Heizelemente bezeichnet, wobei der Kontaktsensor anhand der Korrelation aus Stromfluss und tatsächlicher Temperatur Rückschlüsse über das Vorliegen eines Hautkontaktes ziehen kann.

Es versteht sich, dass Kontaktsensoren im Sinne der Erfindung stets nur wahrscheinliche Aussagen über das Kontaktieren einer Haut treffen können anhand von messbaren Parametern, welche eine Haut insbesondere im Gegensatz zur Luft auszeichnen. Dies kann beispielsweise thermische Eigenschaft Haut als thermische Last, elektrische Eigenschaften der Haut wie z. B. deren Leitfähigkeit und/oder optische Eigenschaften, wie deren Opazität, betreffen. Das Feststellen eines Kontaktes mit der Haut ist in diesem Sinne als bevorzugt als eine Messaussage zu verstehen für ein wahrscheinliches Kontaktieren einer Haut, im Gegensatz zu einem Zustand bei der die Behandlungsfläche an der Luft liegt.

In einer bevorzugten Ausführungsform der Erfindung ist die Vorrichtung dadurch gekennzeichnet, dass der Kontaktsensor ein optischer Detektor ist oder einen solchen umfasst. Im Sinne der Erfindung bezeichnet ein optischer Detektor bevorzugt einen Sensor für elektromagnetische Strahlung, bevorzugt im sichtbaren Bereich, d.h. in einem Wellenlängenbereich von 400 - 700 nm. Ein optischer Detektor für den sichtbaren Bereich wird bevorzugt auch als Lichtsensor bezeichnet. Es kann aber auch sein, dass der optische Detektor elektromagnetische Strahlung im infraroten oder ultravioletten Bereich detektiert. Für die Zwecke der Vorrichtung eignen sich verschiedene optische Detektoren, wie beispielsweise Photozellen, Photomultiplier, CMOS-Sensoren, CCD-Sensoren, Photodioden, Phototransistoren oder Fotowiderständen.

Gemein ist diesen Detektoren, dass diese Veränderungen der Intensität einfallender elektromagnetischer Strahlung bestimmen und zumeist in Form eines elektrischen Signales an die Regelvorrichtung weitergeben können. Bevorzugt wird der optische Detektor innerhalb der Behandlungsfläche oder in unmittelbarer Nähe der Behandlungsfläche installiert vorliegen. Somit wird bei einer Anwendung der Vorrichtung und dem Aufbringen der Behandlungsfläche auf eine Haut der optische Detektor mindestens teilweise, bevorzugt zur Gänze, von der Hautpartie abgedeckt. Hierdurch kommt es zu einer Veränderung der gemessenen Lichtintensität. Anhand der Messdaten über die Veränderung der Lichtintensität kann die Regelungsvorrichtung das Vorliegen eines Kontaktes mit der Haut feststellen. Zu diesem Zweck kann die Regelvorrichtung auch Referenzdaten umfassen, beispielsweise über durchschnittliche Lichtintensitäten bei Umgebungslicht oder über Schwellwerte, ab deren Unterschreitung der optische Detektor mindestens teilweise oder zur Gänze abgedeckt ist.

Die Ausführungsform zeichnet sich durch eine einfache und dennoch zuverlässige Implementation eines Kontaktsensors aus, welche zudem nur mit geringen Zusatzkosten verbunden ist.

In einer weiteren bevorzugten Ausführungsform der Erfindung ist die Vorrichtung dadurch gekennzeichnet, dass der Kontaktsensor ein kapazitiver Sensor ist oder einen solchen umfasst. Ein kapazitiver Sensor bezeichnet im Sinne der Erfindung bevorzugt einen Sensor, welcher die Veränderung der elektrischen Kapazität eines oder mehrerer Kondensatoren erfasst. Der kapazitive Sensor liegt bevorzugt innerhalb der Behandlungsfläche installiert vor. Sobald diese eine menschliche Hautpartie oder Lippe kontaktiert, ändert sich die gemessene Kapazität aufgrund der Einkopplung der Fremdkapazität. Ein kapazitiver Sensor kann somit zuverlässig das Kontaktieren einer Hautpartie anzeigen.

In einer weiteren bevorzugten Ausführungsform der Erfindung ist die Vorrichtung dadurch gekennzeichnet, dass der Kontaktsensor ein taktiler Sensor ist oder einen solchen umfasst. Unter einem taktilen Sensor wird bevorzugt ein Sensor verstanden, welcher auf mechanischer Basis eine Kontaktierung der Behandlungsflache detektieren kann. Das Messprinzip beruht mithin bevorzugt darauf, dass beim Auflegen der Behandlungsfläche auf die Haut ein Druck vermittelt wird, welcher mechanisch mittels des taktilen Sensors detektiert werden kann. Dem Fachmann sind verschiedene geeignete taktile Sensoren bekannt, beispielsweise können diese Messfedern oder piezoelektrische Elemente umfassen, welche ein Eindrücken oder Verschieben der gesamten Behandlungsfläche detektieren.

In einer weiteren bevorzugten Ausführungsform der Erfindung ist die Vorrichtung dadurch gekennzeichnet, dass der Kontaktsensor ein Pyrometer ist oder ein solches umfasst. Im Sinne der Erfindung bezeichnet ein Pyrometer einen Sensor zur berührungslosen Temperaturmessung. Bevorzugt erfolgt die berührungslose Temperaturmessung auf Basis der Messung der Wärmestrahlung, welcher jeder Körper in Abhängigkeit seiner Temperatur emittiert. Das Pyrometer kann daher auch als Strahlungsthermometer oder Infrarotsensor bezeichnet werden. Die Bestimmung der Temperatur eines Körpers hängt von dessen Emissionsgrad ab. Unter dem Emissionsgrad versteht man das Verhältnis der abgestrahlten Leistung eines beliebigen Körpers zur abgestrahlten Leistung eines Schwarzen Strahlers gleicher Temperatur. Der Emissionsgrad ist materialabhängig. Außerdem kann er sich für bestimmte Materialien mit der Wellenlänge, der Temperatur oder anderen physikalischen Größen ändern.

In bevorzugten Ausführungsformen wird das Pyrometer oder der Infrarotstrahler in der Behandlungsfläche versetzt eingelassen, sodass die Temperatur von Objekten, welche sich vor der Behandlungsfläche befinden oder welche die Behandlungsfläche kontaktieren gemessen werden kann. Bei Feststellen einer Temperatur, welcher typischerweise der Oberflächentemperatur auf einer Haut entspricht, kann anhand der gemessenen Daten des Pyrometers eine Kontaktierung der Haut festgestellt werden. Als Temperaturbereiche eignen sich hierzu insbesondere ca. 28-34°C, bevorzugt ca. 30-33°C. Geeignete Wellenlängenbereiche zur Messung dieser Temperaturbereiche sind im mittleren Infrarotbereich, bevorzugt zwischen 3 und 20 µm. Der Fachmann kennt verschiedene Pyrometer, welche für die genannten Zwecke geeignet sind. Insbesondere kann der Fachmann hierbei auch auf bekannte Technologien zur berührungslosen Temperaturmessung zurückgreifen, welche in berührungslosen Fieberthermometern verwandt werden.

In einer bevorzugten Ausführungsform ist die Regelvorrichtung derart konfiguriert ist, dass der Zeitraum der Behandlungsphase in Abhängigkeit davon bestimmt wird, wann ein Kontakt der Behandlungsfläche mit der Haut festgestellt wird. Durch eine Steuerung der Dauer der Behandlungsphase in Abhängigkeit der Kontaktierung der Haut kann ein deutlich präziserer Wärmeübertrag erfolgen. Falls beispielsweise das Aufheizen gestartet wurde und ein Kontakt mit der Haut erst zu einem späteren Zeitpunkt festgestellt wird, kann die Behandlungsphase verlängert werden. Hierdurch kann sichergestellt werden, dass der gewünschte und therapeutische wirksame Wärmeübertrag erfolgt. Durch eine derartige Regulation können wiederholbare Ergebnisse erzielt und abweichendes Verhalten bei der Verwendung der Vorrichtung wirksam ausgeglichen werden.

In einer weiteren bevorzugten Ausführungsform der Erfindung ist die Vorrichtung dadurch gekennzeichnet, dass die Regelvorrichtung derart konfiguriert ist, dass ein Erhitzen des Heizelementes nur initialisiert wird, falls ein Kontakt der Behandlungsfläche mit der Haut festgestellt wird oder ein Erhitzen des Heizelementes abgebrochen wird, sobald festgestellt wird, dass kein Kontakt der Behandlungsfläche mit der Haut vorliegt.

Hierdurch kann ein Aufheizen beispielweise durch zufälliges Betätigen eines Schalters beim Transport der Vorrichtung verhindert werden. Auch ein unnötiges Nachheizen, obwohl der Nutzer bereits wissentlich oder unwissentlich die Behandlung abgebrochen hat, wird vermieden. Dies spart Energie und sichert gegen unsachgemäße Nutzung ab.

In einer weiteren bevorzugten Ausführungsform umfasst die Vorrichtung ein wasserdichtes Gehäuse. Das Gehäuse stellt bevorzugt eine äußere Ummantelung der Vorrichtung dar, sodass diese insbesondere die Regelvorrichtung und anderen elektronische Komponenten umschließt. Es ist bevorzugt, dass das Gehäuse einen Gehäusekopf und einen Gehäusegriff aufweist, wobei die Behandlungsfläche bevorzugt an einem unteren Abschnitt des Gehäusekopfes vorliegt. Zur Steuerung und Temperierung der Behandlungsfläche weist das Gehäuse bevorzugt an der entsprechenden Position einen Durchbruch auf. In der bevorzugten Ausführungsform ist das Gehäuse derart ausgestaltet, dass sämtliche Durchbrüche, d.h. zum Beispiel ebenfalls eventuell vorhandene Batteriefächer, wasserdicht sind. Beispielsweise können zu diesem Zweck Dichtungsringe oder geeignete Dichtungen, zum Beispiel aus Elastomeren, eingesetzt werden. Der Fachmann kennt jedoch viele weitere technische Möglichkeiten, um ein Gehäuse wasserdicht zu konstruieren. Die wasserdichte Ausgestaltung des Gehäuses stellt ein (zusätzliches) Sicherheitselement dar, da hierdurch Schäden an der Regelvorrichtung oder anderen elektronische Komponenten aufgrund von eintretenden Flüssigkeiten wirksam vermieden werden können. Auch führt das wasserdichte Gehäuse zur Vermeidung von Korrosion und somit zu einer verlängerten Lebensdauer der Vorrichtung. Insbesondere in Verbindung mit der Verwendung von Schutzlack kann die Sicherheit synergistisch erhöht werden. Dies spielt insbesondere bei Desinfektionsvorgängen der Vorrichtung und vor allem der Behandlungsfläche eine Rolle. Somit kann die Vorrichtung sehr einfach und fehlerfrei gründlich desinfiziert werden, indem bevorzugt die ganze Vorrichtung in eine Desinfektionsflüssigkeit getaucht bzw. verbracht wird und dort für eine gewisse Mindestdauer verbleibt.

In weiteren bevorzugten Ausführungsformen umfasst die Vorrichtung weitere Sicherheitselemente, welche die Temperatur der Behandlungsfläche kontrollieren.

Zum einen kann die Vorrichtung bevorzugt einen hardwareimplementierten Temperaturwächter umfassen, welcher die Maximaltemperatur der Behandlungsfläche auf einen Wert zwischen 54 °C und 58 °C bevorzugt ungefähr 56 °C begrenzt. Der hardwareimplementierte Temperaturwächter erlaubt vorteilhafterweise eine Sicherstellung, dass eine Maximaltemperatur einen Wert zwischen 54 °C und 58 °C bevorzugt von ungefähr 56°C nicht überschreitet. Der "hardwareimplementierte Temperaturwächter" bezeichnet bevorzugt ein Temperaturkontrollsystem für die Behandlungsoberfläche, welches hardwarebasiert die Stromversorgung der Heizelemente für die Behandlungsfläche abschalten kann. Insbesondere erlaubt der "hardwareimplementierte Temperaturwächter" bevorzugt, dass eine Abschaltung der Stromversorgung der Heizelemente bei Überschreitung der Maximaltemperatur unabhängig von der Regulation der Heizelemente durch die Regelvorrichtung, also z.B. dem Mikroprozessor erfolgt. Sollte zur Regulation der Heizelemente beispielsweise auf der Regelvorrichtung eine Firmware installiert vorliegen, so ist es bevorzugt, dass der hardwareimplementierte Temperaturwächter auch bei einem Ausfall oder einem fehlerhaften Ausführen der Firmware die Maximaltemperatur der Behandlungsfläche zuverlässig begrenzt.

Auch andere, geeignete Maximaltemperaturen, z. B zwischen 43 °C und 47 °C, können bevorzugt sein.

Hierdurch kann durch einfache konstruktive Mittel besonders effektiv sichergestellt werden, dass die Behandlungsfläche der Vorrichtung eine Maximaltemperatur nicht überschreitet. Selbst unter Auftreten von Fehlsteuerungen in der Regelvorrichtung, beispielsweise nach dem Eintreten von Flüssigkeiten, kann aufgrund des hardwarebasierten Temperaturwächters vorteilhafterweise jederzeit gewährleistet werden, dass die Behandlungsfläche eine Maximaltemperatur von einem Wert zwischen 54 °C und 58 °C bevorzugt von ungefähr 56 °C nicht überschreitet. Durch dieses zusätzliche technische Element zur Temperaturüberwachung ist es möglich einen ausgezeichneten Sicherheitsstandard zu wahren, ohne mit der Funktionsweise der Vorrichtung zur hyperthermischen Behandlung zu interferieren.

Als weiteres Sicherheitselement kann die Vorrichtung eine Schmelzsicherung aufweisen, welche bei einem Kurzschluss der Vorrichtung oder ungeregeltem Durchheizen der Vorrichtung die Stromversorgung zur Vorrichtung unterbricht. Im Sinne der Erfindung wird unter einer Schmelzsicherung bevorzugt eine Überstromschutzeinrichtung verstanden, bei welcher beispielsweise durch das Abschmelzen eines Schmelzleiters ein Stromkreis unterbrochen werden kann, sobald die Stromstärke einen Grenzwert über eine zu bestimmende Zeit überschreitet. Es ist bevorzugt, dass die Schmelzsicherung in der Vorrichtung zwischen der Einspeisung der Versorgungsspannung in die Vorrichtung und der Vorrichtung selbst vorliegt. Sollte es zu einem Störfall kommen, welcher dadurch gekennzeichnet ist, dass ein unkontrollierter hoher Strom von der Versorgungseinspeisung in die Vorrichtung fließt, schaltet die Schmelzsicherung vorteilhafterweise die komplette Spannungsversorgung der Vorrichtung ab. Eine Schmelzsicherung bietet einen ausreichend schnellen und extrem zuverlässigen Schutz.

Es hat sich gezeigt, dass selbst unter fehlerfreier Konstruktion der Vorrichtung und dem Bereitstellen eines hardwareimplementierten Temperaturwächters es nicht ausgeschlossen werden kann, dass es aufgrund einer fehlerhaften Bedienung oder Schädigung der Vorrichtung, in äußerst seltenen Fällen zu einem Durchheizen der Heizelemente kommt. Unter dem Durchheizen der Heizelemente wird im Sinne der Erfindung bevorzugt verstanden, dass die Temperatur der Heizelemente unkontrolliert, d.h. nicht durch eine temperaturbasierte Regelung mithilfe der Regelvorrichtung steigt. Falls in diesen Störfällen der hardwareimplementierte Temperaturwächter versagt, kann die Behandlungsfläche unkontrolliert auf Temperaturen weit über der gewünschten Kontakttemperatur ansteigen, beispielsweise auf Temperaturen von weit über 65 °C.

Obwohl ein solches fehlerhaftes Durchheizen äußerst selten auftritt, kann dies zu Schädigungen bei den Probanden führen. Dies liegt insbesondere daran, dass die hyperthermisch zu behandelnden Hautpartien, wie z. B. die Lippen, zumeist besonders empfindlich sind und beispielsweise durch eine Rötung, Schwellung oder gar Wundbildung gekennzeichnet sind. Eine deutlich erhöhte Temperatur über 65° kann lokal an diesen Stellen in besonderen Maße zu starken Schmerzen und Hautverbrennungen führen.

Die Schmelzsicherung ist somit geeignet, um noch für den unwahrscheinlichsten Fall einer Störung eine Abschaltung des Heizens der Behandlungsfläche gewährleisten zu können. So wird mithilfe der Schmelzsicherung unabhängig von jeglicher Temperaturmessung, aufgrund z.B. fehlerhafter Temperatursensoren, ein übermäßiges Heizen der Behandlungsfläche unterbunden. Vorteilhafterweise stellt Stromversorgung der Vorrichtung eine zentrale Regulationsschnittstelle dar, welche höchsten Sicherheitsanforderungen genügt. Durch eine Integration der Schmelzsicherung in den Stromfluss zur Versorgung der Vorrichtung kann sichergestellt werden, dass ein maximaler Versorgungsstrom während einer bestimmten Zeit nicht überschritten wird. Da ein Durchheizen und ein unkontrolliertes Heizen der Heizelemente über die gewünschte Temperatur mit einem erhöhten Stromfluss zusammenhängt, kann hierdurch auf besonders zuverlässige Weise ein Überhitzen der Behandlungsfläche vermieden werden. Insbesondere kann durch die Stromkontrolle sehr schnell reagiert werden, bevor der Strom so lange wirkt, dass er eine seiner Stärke entsprechende Temperatur erzeugt.

Besonders vorteilhaft ist die kombinierte Verwendung eines hardwareimplementierten Temperaturwächters und einer Schmelzsicherung.

So ist es ein Nachteil der Schmelzsicherung, dass diese nach dem einmaligen Auslösen eine permanente Abkopplung der Versorgungsspannung von der Vorrichtung zur Folge hat. Eine erneute Inbetriebnahme der Vorrichtung nach dem Auslösen der Schmelzsicherung bedarf der Reparatur durch einen Techniker, beispielsweise den Austausch der Schmelzsicherung. Im Hinblick auf die Kosten ist die Vorrichtung in der Regel bei einem Auslösen der Schmelzsicherung unbrauchbar geworden.

Vorteilhafterweise ist der hardwareimplementierte Temperaturwächter jedoch so eingestellt, dass es nicht zu einem permanenten Abschalten der Stromversorgung der Vorrichtung kommen muss. Vielmehr ist der hardwareimplementierte Temperaturwächter derart konzipiert, dass wenn die Temperatur der Behandlungsfläche eine Maximaltemperatur überschreitet, während des Zeitraumes des Überschreitens die Stromversorgung der Heizelemente unterbrochen wird. Die Stromunterbrechung durch den hardwareimplementierten Temperaturwächter ist somit vorteilhafterweise reversibel, d.h. sobald die Temperatur der Behandlungsfläche wieder unter die Maximaltemperatur fällt, können die Heizelemente wieder heizen.

So kann auch nach einem einmaligen Auftreten einer Fehlsteuerung die bestimmungsgemäße Benutzung der Vorrichtung fortgeführt werden. Der Benutzer würde gegebenenfalls nichts von der Fehlsteuerung bemerken, da durch die gewählte Maximaltemperatur, die Effektivität und die Unabhängigkeit des hardwareimplementierten Temperaturwächters keine für den Benutzer als unangenehm empfundenen Temperaturen entstehen und die Vorrichtung bei der nächsten Benutzung im Falle einer einmaligen Fehlsteuerung wieder einwandfrei funktioniert könnte.

Die Kombination der Sicherheitsmerkmale eines hardwareimplementierten Temperaturwächters mit einer Schmelzsicherung erlauben eine überraschend zuverlässige Kontrolle der Temperatur mit möglichst wirtschaftlichen Mitteln aufgrund gestaffelter Sicherheitsschranken.

In einer bevorzugten Ausführung der Erfindung umfasst der hardwareimplementierte Temperaturwächter mindestens einen zweiten Temperatursensor zur Messung der Temperatur der Behandlungsfläche und einen Komparator, wobei der Komparator die Temperatur der Behandlungsfläche mit der Maximaltemperatur vergleicht und bei Überschreiten der Maximaltemperatur die Stromzufuhr zu dem mindestens einen Heizelement unterbindet. Im Sinne der Erfindung bezeichnet ein Komparator bevorzugt eine elektronische Schaltung zum Vergleichen zweier Spannungen, wobei am Ausgang in binärer Weise angezeigt wird, welcher der beiden Spannungen höher ist. Im Stand der Technik sind hinreichend verschiedene Komparatoren bekannt, welche geeignet sind aus zwei analogen Spannungen ein binäres Ausgangsignal auszugeben, welche aussagt, welche der Eingangsspannung höher ist. Als Beispiel für eine Komparator-Schaltung sei der Schmitt-Trigger genannt. Es ist bevorzugt, dass an einem Eingang des Komparators mit Hilfe eines Spannungsverteilers ein Referenzwert für eine Spannung angelegt wird. Dieser Referenzwert entspricht bevorzugt dem Spannungswert, welcher der zweite Temperatursensor aufweisen würde, wenn die Temperatur der Behandlungsfläche gleich der Maximaltemperatur ist. An dem zweiten Eingang des Komparators liegt bevorzugt die Ausgangsspannung des Temperatursensors an, welche von der Temperatur der Behandlungsfläche abhängt. Ein besonders bevorzugter Temperatursensor umfasst zu diesem Zweck einen NTC-Thermistor, d.h. einen Heißleiter. Dieser besitzt einen negativen Temperaturkoeffizienten, sodass bei einer Erhöhung der Temperatur der Widerstand fällt und ein höherer Strom fließt. Es können aber auch Kaltleiter, d.h. PTC-Thermistoren verwandt werden, welche einen positiven Temperaturkoeffizienten besitzen, sodass bei einer Erhöhung der Temperatur der Widerstand steigt und ein niedrigerer Strom fließt.

Steigt die Temperatur der Behandlungsfläche, bewegt sich der durch den zweiten Temperatursensor gesteuerte Spannungswert am Komparator auf den Referenzwert der Spannung zu, welcher der Maximaltemperatur entspricht. Sobald die Temperatur die Maximaltemperatur überschreitet, ändert sich das Ausgangsignal am Komparator binär. Der Komparator ist bevorzugt in die Stromversorgung der Heizelemente integriert. D.h. bevor die Temperatur der Behandlungsfläche die Maximaltemperatur erreicht, gibt der Komparator bevorzugt die Versorgungsspannung der Heizelemente frei. Sobald jedoch die Temperatur höher als die Maximaltemperatur ist, schaltet der Ausgang des Komparators ab und unterbricht die Versorgungsspannung der Heizelemente. Fällt die Temperatur der Behandlungsfläche wieder, wird die Versorgungsspannung vorteilhafterweise wieder durch den Komparator freigegeben. Hierdurch kann ein reversibles An- und Abschalten der Heizelemente nur für den Zeitraum erfolgen, während die Temperatur der Behandlungsfläche die Maximaltemperatur überschreitet. Weiterhin kann es bevorzugt sein, dass der Komparator durch die Regelvorrichtung beim Starten der Vorrichtung freigeschaltet wird. Erfolgt somit kein ordnungsgemäßer Start der Vorrichtung ist der Komparator in der Voreinstellung so konfiguriert, dass die Spannungsversorgung der Heizelemente unterbrochen ist.

Die beschriebene bevorzugte Ausführungsform des hardwareimplementierten Temperaturwächters hat sich in Tests als besonders robust und zuverlässig erwiesen. Aufgrund der Reversibilität der Sicherheitsabschaltung und einfachen Konstruktionsweise zeichnet sich die bevorzugte Ausführungsform weiterhin durch niedrige Herstellungs- und Wartungskosten aus.

Durch die von der Regelvorrichtung unabhängigen Bauweise samt eigenem Temperatursensor kann ein zuverlässiger Betrieb auch bei Ausfall eines Bauteils der Regelvorrichtung gewährleistet werden.

Ebenso ist ein hardwareimplementierter Temperaturwächter in beschriebener Form unter Ausnutzung eines Komparators besonders schnell, da Komparatoren weit verbreitete elektronische Bauteile sind, die sich neben ihrer Zuverlässigkeit durch ihre schnelle Schaltfähigkeit ausweisen. So gibt es beispielsweise Komparatoren mit Schaltzeiten von ns und darunter.

In einer bevorzugten Ausführungsform der Erfindung ist die Vorrichtung dadurch gekennzeichnet, dass die Schmelzsicherung einen Schwellenwert für einen maximalen Strom aufweist, welcher der Erhitzung der Behandlungsfläche auf einen Wert zwischen 65 °C und 70 °C bevorzugt von 65 °C für 1 Sekunde entspricht. Tests haben gezeigt, dass erst eine Temperaturerhöhung von über 65 °C für länger als 1 Sekunde sehr kritisch für das Schmerzempfinden ist und zu Schädigungen der Hautpartien führen kann. Vorteilhafterweise wird durch Einstellung der Schmelzsicherung auf diese Parameterwerte die Schmelzsicherung nicht vorzeitig bei unkritischen Temperaturerhöhungen der Behandlungsfläche ausgelöst. Hierdurch kann Wirtschaftlichkeit erhöht werden, ohne einen Kompromiss in Bezug auf die Sicherheit einzugehen. Der Fachmann weiß anhand der elektrischen Parameter der Heizelemente, welche Schmelzsicherung gewählt werden sollte, um die angebenden Werte zu gewährleisten. Hierzu kann auch eine Messung der Stromzufuhr bei gleichzeitiger Messung der Temperatur der Behandlungsfläche erfolgen. Weiterhin ist es besonders bevorzugt eine flinke Schmelzsicherung zu verwenden, welche auf eine Stromerhöhung innerhalb von bevorzugt weniger als 20 ms reagiert. So wurde erkannt, dass auch eine kurzfristige Stromerhöhung von weniger als 20 ms aufgrund der thermischen Trägheit der Behandlungsfläche zu einer Temperaturerhöhung von länger als 1 Sekunde führen kann.

Gegenüber nicht rückstellenden, rein temperaturabhängigen Thermosicherungen, hat eine stromabhängige Schmelzsicherung einige Vorteile. Bei nicht rückstellenden, reinen temperaturabhängigen Thermosicherungen geschieht das Abschmelzen nicht bei Anliegen eines Stromes oberhalb eines Schwellenwertes, sondern erst bei Anliegen einer externen Temperatur, welche größer ist als eine definierte Maximaltemperatur. Gegenüber nicht rückstellenden reinen temperaturabhängigen Thermosicherungen können stromabhängige Schmelzsicherungen somit schon reagieren, bevor in Folge eines länger wirkenden erhöhten Stroms eine bestimmte unerwünschte Temperatur überhaupt erreicht wird. Ebenso brauchen nicht rückstellende reine temperaturabhängige Thermosicherungen bei Anliegen einer externen Temperatur, welche größer ist als eine definierte Maximaltemperatur, immer eine gewisse Reaktionszeit. Somit kann es zu gefährlichen, weiteren Temperaturerhöhungen kommen. Stromabhängige Schmelzsicherungen reagieren im Gegensatz dazu schneller und mit minimalen systembedingten Latenzzeiten.

In einer bevorzugten Ausführungsform der Erfindung ist die Vorrichtung dadurch gekennzeichnet, dass der Schwellenwert der Schmelzsicherung bevorzugt zwischen 1 A und 2,5 A besonders bevorzugt ungefähr 2 A beträgt. Tests haben gezeigt, dass in Bezug auf die bevorzugten Heizelemente die genannten Schwellenwerte besonders zuverlässig gewährleisten, dass die Temperatur der Behandlungsfläche eine Temperatur von 65 °C bis 70 °C für nicht länger als 1 Sekunde überschreitet. Durch das Schmelzen der Schmelzsicherung ab Stromwerten von 1 A bis 2.5 A kann somit sichergestellt werden, dass die Temperatur der Behandlungsfläche nicht in einen gesundheitsgefährdenden Bereich kommen kann. So kommt es bei einer regulären Behandlung zu einem regulären Behandlungsstrom, welcher unterhalb von 2,5 A, bevorzugt 1 A liegt. Tritt ein Fehler auf, z.B. bei einem Durchheizen, so fließt ein erhöhter Strom. In diese Falle Sicherung greift die Sicherung ein und verhindert wirksam ein unkontrolliertes Aufheizen.

Es kann ebenso bevorzugt sein, nur eines der Sicherheitselemente ausgewählt aus hardwareimplementierter Temperaturwächter und/oder Schmelzsicherung zu verwenden. So kann ein besonders einfacher Aufbau realisiert werden, wobei ein akzeptables Sicherheitsniveau erreicht wird.

In einer bevorzugten Ausführungsform der Erfindung beträgt die Kontakttemperatur zwischen 43 °C und 56 °C. So können verschiedene Leiden sehr individuell behandelt werden. Insbesondere wurde erkannt, dass eine besonders starke Überlagerung des Juckempfindens erreicht werden kann, wenn in den betreffenden Hautpartien lokal die Thermo- und Capsaicinrezeptoren TRPV1 und TRPV2 zeitgleich aktiviert werden. Der TRPV1 ist bei akutem Hitze-induzierten Schmerz in gesunder Haut beteiligt und reguliert beispielsweise das Heißempfinden bei Temperaturen um 45° bis 50 °C. Bei besonders starken schmerzhaften Hitzereizen, welche ab Temperaturen von über 52 °C eintreten wird darüber hinaus der TRPV2 aktiviert. Die Aktivierungsschwelle des TRPV1 liegt zwischen 40°C und 45°C, wohingegen die des TRPV2 zwischen 50°C und 53° beträgt (Yao et al 2011, Somogyi et al. 2015, Cohen et al. 2014, Mergler et al. 2014). Während durch aktuelle Forschungsergebnisse in der Literatur ein erstes Verständnis über die Wirkungsweise der TRPV1 und TRPV2 Rezeptoren als Temperatursensoren aufkommt, ist deren Rolle in der Empfindung von Juckreizen unbekannt. Ein Fachmann würde daher auch unter Kenntnis der Literatur nicht davon ausgehen, dass gerade die Aktivierung dieser Rezeptoren eine besonders effektive Überlagerung des Juckempfindens ermöglicht.

In einer bevorzugten Ausführungsform der Erfindung beträgt die Kontakttemperatur 43-47°C, wobei die Regelvorrichtung konfiguriert ist, die Kontakttemperatur für einen Zeitraum zwischen 1 bis 10 Sekunden zu halten.

Durch die erfindungsgemäße präzise Regulation der Kontakttemperatur in einem Bereich von 43-47°C für die Behandlungsphase von 1 - 10 s konnten sehr gute Ergebnisse in Bezug auf einen Rückgang von Juckreiz und/oder Herpesbläschen innerhalb von kürzester Zeit erreicht werden, ohne dass über stechenden Schmerz geklagt wurde. Die Compliance und der Therapieerfolg waren für die erfindungsgemäße Vorrichtung durchweg gut.

Ausgezeichnete Ergebnisse konnten mit einer bevorzugten Aufführungsform der Vorrichtung erzielt werden, bei welche die Kontakttemperatur zwischen 44,5 °C - 46,5 °C, besonders bevorzugt zwischen 45°C - 46°C beträgt. Für die vorgenannten Temperaturbereiche konnte in Studien teilweise ein sicht- und/oder fühlbares Abklingen von Juckreiz und/oder Herpesbläschen und Rötungen innerhalb von 2 Tagen, teilweise innerhalb eines Tages verzeichnet werden. Dies deutet darauf hin, dass die vorgenannten Bereiche ein optimales Behandlungsregime darstellen. Der hohe therapeutische Erfolg des Behandlungsregimes kann nur teilweise z. B. durch die Thermolabilität des DNA-bindenden Proteins des Herpesvirus erklärt werden. Gleichzeitig scheint vielmehr für die genannten Temperaturbereiche von 44,5 °C - 46,5 °C, besonders von 45°C - 46°C zudem das körpereigene Immunsystem unterstützt zu werden, sodass in Bezug auf den Behandlungserfolg für den engen Temperaturbereich eine synergistische Wirkung verantwortlich ist, welche eine Hemmung der Replikation der Herpesviren und/oder eine thermischen Neutralisation der Gifte bspw. von Insekten mit gleichzeitiger Aktivierung bzw. Unterstützung des körpereigenen Immunsystems umfasst.

Zudem berichteten Probanden für die bevorzugten Kontakttemperaturen von 44,5 °C - 46,5 °C, besonders bevorzugt zwischen 45°C - 46°C von einem deutlich verminderten Juckreiz. Das Abmildern des Juckempfindens hielt überraschenderweise noch Stunden nach der Behandlung an. Als sekundärer Behandlungseffekt konnte ein vermindertes Aufkratzen von Herpesbläschen bei der Behandlung von Herpes verzeichnet werden, welches zusätzlich zu einer schnelleren Heilung beiträgt.

Dies traf insbesondere auf die Kombination der vorgenannten Temperaturen mit einer Behandlungsdauer von 2 bis 5 Sekunden zu.

In einer bevorzugten Ausführungsform der Erfindung beträgt die Aufheizphase 1 Sekunde bis 5 Sekunden, bevorzugt weniger als 3 Sekunden und insbesondere 1 bis 2 Sekunden. Durch so eine schnelle Aufheizphase kann besonders schnell die gewünschte Temperatur erreicht werden. Somit können bevorzugt Heilungseffekte erzielt werden, ohne einem Anwender unnötig Hitze zuzuführen und/oder die effektiv für eine Behandlung benötigte Zeit zu vergrößern. Außerdem kann so die abgegebene Wärmemenge während der Behandlung besonders präzise bestimmt werden.

In einer besonders bevorzugten Ausführungsform der Erfindung ist die Größe der Behandlungsfläche zwischen 30 mm² und 50 mm². Insbesondere bei Herpeserkrankungen, vor allem am Mund (sogenanntes Herpes *labialis*), ist die bevorzugte Größe der Behandlungsfläche ideal, um alle möglichen betroffenen Stellen abzudecken. Insbesondere eine Behandlungsfläche zwischen 30 und 50 mm² ist geeignet, alle typischen betroffenen Hautpartien bei nur einmaligem Auflegen der Vorrichtung abzudecken. Aber auch zur Behandlung von Insektenstichen, z. B. von Mücken oder Ameisen, kann diese Größe besonders geeignet sein. Des Weiteren kann eine Vorrichtung, welche eine solche Behandlungsfläche aufweist, besonders kompakt gehalten werden. So können Vorrichtungsgrößen, welche der eines Lippenstifts entsprechen, erreicht werden. Ein solch kompaktes Gerät wird gern und bereitwillig dauerhaft am Körper oder in einer mitgeführten Tasche getragen, sodass eine Behandlung jederzeit vorgenommen werden kann. Dies steigert den Behandlungserfolg erheblich. Die Behandlungsfläche ist bevorzugt rund, dies eignet sich besonders zur Behandlung von Herpes, dessen betroffene Hautpartien oftmals annähernd runde Form aufweisen.

Es können auch beliebige, dreidimensionale Formen, insbesondere konvexe Formen verwendet werden, welche sich für die Behandlung von Herpes besonders eignen. Z. B. kann die Form eines Lippenstifts verwendet werden, wobei der Benutzer bei der Behandlung zu einem leichten Aufdrücken der Vorrichtung animiert wird. Hierdurch kann ein psychologischer Effekt ausgelöst werden, der ein Wohlbefinden bei der Behandlung verstärkt. Auch kann die übertragene Wärmemenge verbessert werden. Es kann eine organische Form verwendet werden, welche sich zur Behandlung insbesondere der Lippen besonders gut eignet.

Zudem zeigt sich, dass eine Kombination der Größe der Behandlungsfläche zusammen mit den bevorzugt genannten Kontakttemperaturen und Behandlungsphase besonders effektiv ist um z. B. Herpes, insbesondere auf Lippenpartien, zu behandeln. Einerseits ist die Behandlungsfläche groß genug, um auch angrenzende Bereiche wirksam abzudecken und so auch den Grenzbereich infizierter Hautpartien wirksam zu behandeln. Anderseits wird die maximale Größe der Behandlungsfläche trotz der hyperthermischen Behandlung als besonders angenehm ohne jegliche schmerzhaften Empfindungen wahrgenommen. Dies ist insbesondere im empfindlichen Bereich der Mundpartie, vor allem der Lippen, von besonderer Bedeutung. Für Probanden mit den vorgenannten Parametern werden sowohl die besten Compliancewerte, also die höchste Bereitschaft der Patienten zur aktiven Mitwirkung und Anwendung der Vorrichtung, als auch die besten Therapieerfolge verzeichnet.

In einer weiteren bevorzugten Ausführungsform der Erfindung beträgt die Größe der Behandlungsfläche zwischen eine Größe der Behandlungsfläche zwischen 6 cm² und 18 cm². So kann Juckreiz insbesondere auf großflächigen Hautpartien besonders stark reduziert werden kann

In einer weiteren bevorzugten Ausführungsform der Erfindung ist die Vorrichtung dadurch gekennzeichnet, dass die Vorrichtung eine Stromversorgungseinheit umfasst sowie einen Spannungswächter, welcher die Spannung der Stromversorgungseinheit überwacht. Im Sinne der Erfindung stellt die Stromversorgungseinheit bevorzugt die elektrische Energie zum Betreiben der Vorrichtung bereit. Bevorzugte Stromversorgungseinheiten sind Batterien oder Akkus. Diese stellen die elektrische Energie zumeist durch Bereitstellung einer Gleichspannung zur Verfügung. In der bevorzugten Ausführungsform wird die durch die Stromversorgungseinheit bereitgestellte Spannung mit Hilfe eines Spannungswächters überwacht. Im Sinne der Erfindung bezeichnet ein Spannungswächter bevorzugt eine elektrische Schaltung, welche die Spannung der Stromversorgungseinheit messen kann und eine Aktion auslöst, wenn diese unter einen vorgegebenen Grenzwert fällt. Im Stand der Technik sind eine Vielzahl von Varianten für Spannungswächter bekannt, wobei der Fachmann weiß, welcher Spannungswächter für welche Arten von Stromversorgungseinheiten, d.h. insbesondere Batterien oder Akkus, geeignet ist. Es ist bevorzugt, dass falls der Spannungswächter einen Abfall der Spannung der Stromversorgungseinheit unter einen bestimmten Wert feststellt, dieser eine Unterbrechungsaufforderung (engl. *interrupt request, IRQ*) an die Regelvorrichtung sendet, welche bevorzugt ein Mikroprozessor ist. Falls währenddessen ein Behandlungszyklus, d.h. ein Aufheizen oder eine Behandlungsphase, durchlaufen wird, führt der *interrupt request* zu einem Abbruch des Behandlungszyklus. Dies stellt einen weiteren Sicherheitsmechanismus dar. So wurde festgestellt, dass eine Unterspannung an der Stromversorgungseinheit zu einem Ausfall der Regelvorrichtung, z.B. des Mikroprozessor führen kann. In dem Fall kann es dazu kommen, dass die Temperaturregulation der Kontakttemperatur mit Hilfe der Regelvorrichtung fehlerhaft ausgeführt wird und es zu einem unkontrollierten Erhitzen der Behandlungsfläche kommt. Der Spannungswächter kann somit zusätzlich dazu beitragen die Sicherheit der Vorrichtung zu erhöhen und eine Gesundheitsgefährdung im Falle beispielsweise einer fehlerhaften Batterie zu vermeiden.

In einer bevorzugten Ausführungsform der Erfindung ist die Vorrichtung dadurch gekennzeichnet, dass die Vorrichtung einen Datenspeicher zur Speicherung der Systemdaten und/oder Fehlermeldungen umfasst. Zu den bevorzugten Systemdaten gehört ein Zähler für die Behandlungszyklen, welcher bevorzugt separat die Verwendung verschiedene Typen von Behandlungszyklen zählt. Kann beispielsweise ein kurzer oder ein langer Behandlungszyklus ausgewählt werden, so wird diese separate gezählt. Weiterhin umfassen die Systemdaten bevorzugt einen Boot Zähler, also eine Zähler dafür wie oft die Vorrichtung gestartet wurde sowie eine Angabe der Fehlermeldungen mit aktuellem Fehlerstand.

Bevorzugt können folgende Fehlermeldungen gespeichert: Ein "Reset" zeigt an, dass der Spannungswächter einen Reset ausgelöst hat. Ein "Watchdog" zeigt an, dass in der Firmware ein Watchdog-Reset aufgetreten ist, d.h. ein Systemneustart aufgrund eines Softwarefehlers. Bevorzugt kann für die Fehlermeldung festgestellt werden in welchem Programmmodus sich das Gerät bei dem Auftreten des Fehlers befand. Ein "Temperatur zu hoch" kann anzeigen, dass die am Temperatursensor gemessene Temperatur zu hoch ist, oder dass der Temperatursensor defekt ist. Ein "Temperatur zu niedrig" kann anzeigen, dass die am Temperatursensor gemessene Temperatur zu niedrig ist, oder dass der Temperatursensor defekt ist. Ein "Kontakttemperatur erreicht" kann anzeigen, ob die gewünschte Kontakttemperatur erreicht wurde oder ein Fehler während der Vorheizphase aufgetreten ist.

Vorteilhafterweise können die gespeicherten Systemdaten und Fehlermeldungen für die Diagnose und Problembehandlung für die Vorrichtung eingesetzt werden. So können diese Daten beispielsweise ausgelesen werden, wenn ein Kunde ein defektes Gerät einsendet. Anhand der Daten ist es möglich den aufgetretenen Fehler, z.B. "Temperatur zu hoch", mit weiteren Systemdaten zur Anzahl der Behandlungszyklen oder Watchdog-Resets zu korrelieren. Anhand dieser Daten kann somit sowohl während der Entwicklungsphase, als auch danach, die Sicherheitsmerkmale der Vorrichtung kontinuierlich optimiert werden. Die Möglichkeit, dass die Vorrichtung eine Speicherung von Systemdaten und Fehlermeldungen umfasst, erlaubt somit die kontinuierliche Verbesserung der Hardware- sowie Softwarebestandteile der Vorrichtung anhand aussagekräftiger Daten.

In einer weiteren bevorzugten Ausführungsform ist die Vorrichtung dadurch gekennzeichnet, dass auf der Regelvorrichtung einer Firmware installiert vorliegt, welche mindestens die Temperaturregulation der Behandlungsfläche steuert, wobei die Firmware einen watchdog counter (WDC) umfasst, welcher überwacht, ob die Firmware ausgeführt wird. Im Sinne der Erfindung wird bevorzugt unter der Firmware eine Software, d.h. die Anleitung für ein computer-implementiertes Verfahren verstanden, welches in der Regelvorrichtung bevorzugt in dem Mikroprozessor eingebettet vorliegt. D.h. die Firmware umfasst bevorzugt jene Software, welche mit der Hardware der Vorrichtung, d.h. insbesondere mit den Heizelementen und Temperatursensoren funktional verbunden ist. Vorzugsweise wird die Firmware mit dem Start der Vorrichtung ausgeführt und übernimmt die Kontroll- und Steuerungsfunktion dieser Hardware-Komponenten der Vorrichtung. Somit wertet die Regelvorrichtung bevorzugt auf Basis der Firmware z.B. die Messdaten der Temperatursensoren sowie Nutzereingaben aus, um während des Behandlungszyklus die Stromzufuhr für die Heizelemente zu steuern. Im Sinne der Erfindung bezeichnen hardwareimplementierte Bauelemente bevorzugt Bauelemente, deren Funktion unabhängig von einer korrekten Ausführung der Firmware sichergestellt ist. Wie obig beschrieben ist der Temperaturwächter hardwareimplementiert, sodass dessen Funktion, d.h. eine Begrenzung der Maximaltemperatur, unabhängig von einer korrekten Ausführung der Firmware auf der Regelvorrichtung erfolgen kann. Selbst bei einem Systemabsturz der Firmware kann der hardwareimplementierte Temperaturwächter daher schnell und korrekt die Maximaltemperatur der Behandlungsfläche begrenzen.

In der besonders bevorzugten Ausführungsform wird die Firmware der Regelvorrichtung mit Hilfe eines hardwareimplementierten Watchdog Counters überwacht. Besonders bevorzugt handelt es sich dabei um einen Time-out-Watchdog. Bevorzugt wird der Time-Out-Watchdog vor dem Start der Behandlungsphase durch die Firmware aktiviert. Während der Behandlungsphase wird von der Firmware innerhalb eines vorbestimmten Zeitintervalls ein Signal an den Time-Out-Watchdog gesendet, um diesen zurückzusetzen. Falls der Time-Out-Watchdog nicht zurückgesetzt wird, führt dies bevorzugt zu einem Neustart der Firmware. Das Zeitintervall richtet sich bevorzugt an die Zeit, welche vorgesehen ist um eine Temperaturmessung und Regelung der Heizelemente durch die Firmware durchzuführen und kann z.B. zwischen 2 ms und 10 ms betragen. Durch einen solchen Time-Out-Watchdog kann vorteilhafterweise sichergestellt werden, dass mindestens während der Behandlungsphase der Vorrichtung die Firmware korrekt funktioniert und die Temperatur der Behandlungsfläche überwacht wird. Durch einen hardwareimplementierten Watchdog zur Überwachung der Firmware, bevorzugt zum Beispiel mit Hilfe eines Time-Out-Watchdogs, kann somit sichergestellt werden, dass falls die Firmware nicht korrekt funktioniert und das vorgegebene Zeitintervall nicht eingehalten wird, die Behandlungsphase abgebrochen wird. Somit ist ein weiteres Sicherheitsfeature der Vorrichtung zusätzlich zu den oben erwähnten vorhanden, welches insbesondere im Zusammenspiel mit dem hardwareimplementierten Temperaturwächter dafür sorgt, dass auch bei nicht korrekt funktionierender Firmware ein Überhitzen der Behandlungsfläche ausgeschlossen ist.

## Patentansprüche

1. Vorrichtung zur Behandlung von Juckreiz und/oder Herpes auf einer Haut, umfassend
a) mindestens eine Behandlungsfläche und
b) eine Regelvorrichtung zur Regulation der Temperatur der Behandlungsfläche,
**dadurch gekennzeichnet, dass**
die Regelvorrichtung konfiguriert ist, die Behandlungsfläche auf einer der Haut zugewandten Außenseite durch Erhitzen mindestens eines Heizelementes in einer Aufheizphase auf eine Kontakttemperatur während eines Kontaktes der Behandlungsfläche mit der Haut zu regulieren und in einer Behandlungsphase zu halten und wobei die Vorrichtung mindestens einen Kontaktsensor umfasst.

2. Vorrichtung gemäß einem der vorherigen Ansprüche
**dadurch gekennzeichnet, dass**
der Kontaktsensor realisiert ist durch mindestens einen Temperatursensor zur Messung der Temperatur der Behandlungsfläche während des Kontaktes mit der Haut und der Regelvorrichtung, welche basierend auf den Messdaten des Temperatursensors das mindestens eine Heizelement reguliert, wobei die Regelvorrichtung auf Basis einer Korrelation der Messdaten des Temperatursensors und Daten über die Ansteuerung des Heizelementes feststellen kann, ob die Behandlungsfläche in Kontakt mit der Haut vorliegt.

3. Vorrichtung gemäß dem vorherigen Anspruch
**dadurch gekennzeichnet, dass**
die Regelvorrichtung Referenzdaten umfasst, über eine Korrelation der Temperatur der Behandlungsfläche mit der Ansteuerung des Heizelementes im Falle, dass die Behandlungsfläche in Kontakt mit der Haut oder mit Luft vorliegt.

4. Vorrichtung gemäß einem der vorherigen Ansprüche **2-3**
**dadurch gekennzeichnet, dass** der Temperatursensor an der Innenseite der Behandlungsfläche vorliegt und die Behandlungsfläche durch eine Keramikschicht mit einer Schichtdicke zwischen 50 µm und 2000 µm gebildet wird und/oder der Temperatursensor an der Innenfläche der Behandlungsfläche vorliegt und die Behandlungsfläche durch eine Goldschicht mit einer Schichtdicke zwischen 50 µm und 2000 µm gebildet wird.

5. Vorrichtung gemäß einem der vorherigen Ansprüche **2-4**
**dadurch gekennzeichnet, dass**
die Behandlungsfläche durch eine Keramik gebildet wird und der Temperatursensor in der Behandlungsfläche integriert vorliegt.

6. Vorrichtung gemäß einem der vorherigen Ansprüche
**dadurch gekennzeichnet, dass**
der Kontaktsensor einen optischen Detektor, einen kapazitiven Sensor, einen taktilen Sensor und/oder ein Pyrometer umfasst.

7. Vorrichtung gemäß einem der vorherigen Ansprüche
**dadurch gekennzeichnet, dass**
die Regelvorrichtung derart konfiguriert ist, dass der Zeitraum der Behandlungsphase in Abhängigkeit davon bestimmt wird, wann ein Kontakt der Behandlungsfläche mit der Haut festgestellt wird.

8. Vorrichtung gemäß einem der vorherigen Ansprüche
**dadurch gekennzeichnet, dass**
die Regelvorrichtung derart konfiguriert ist, dass ein Erhitzen des Heizelementes nur initialisiert wird, falls ein Kontakt der Behandlungsfläche mit der Haut festgestellt wird oder ein Erhitzen des Heizelementes abgebrochen wird, sobald festgestellt wird, dass kein Kontakt der Behandlungsfläche mit der Haut vorliegt.

9. Vorrichtung gemäß einem der vorherigen Ansprüche
**dadurch gekennzeichnet, dass**
die Vorrichtung ein wasserdichtes Gehäuse umfasst.

10. Vorrichtung gemäß einem der vorherigen Ansprüche,
**dadurch gekennzeichnet, dass**
ein hardwareimplementierter Temperaturwächter eine Maximaltemperatur der Behandlungsfläche auf einen Wert zwischen 54 °C und 58 °C, bevorzugt ungefähr 56 °C begrenzt und/oder eine Schmelzsicherung bei Kurzschluss oder ungeregeltem Durchheizen die Vorrichtung abschaltet.

11. Vorrichtung gemäß einem der vorherigen Anspruch
**dadurch gekennzeichnet, dass**
die Kontakttemperatur zwischen 43 °C und 56 °C beträgt.

12. Vorrichtung gemäß einem der vorherigen Ansprüche,
**dadurch gekennzeichnet, dass**
die Kontakttemperatur zwischen 43 °C und 47 °C beträgt und die und die Regelvorrichtung konfiguriert ist, die Kontakttemperatur für einen Zeitraum zwischen 1 bis 10 Sekunden zu halten.

13. Vorrichtung gemäß einem der vorherigen Anspruch
**dadurch gekennzeichnet, dass**
die Kontakttemperatur zwischen 44,5 °C - 46,5 °C, bevorzugt zwischen 45°C - 46°C beträgt und die Regelvorrichtung konfiguriert ist, die Kontakttemperatur für einen Zeitraum zwischen 2 bis 5 Sekunden zu halten.

14. Vorrichtung gemäß einem der vorherigen Ansprüche
**dadurch gekennzeichnet, dass**
die Aufheizphase 1 Sekunde bis 5 Sekunden, bevorzugt weniger als 3 Sekunden und insbesondere 1 bis 2 Sekunden beträgt.

15. Vorrichtung gemäß einem der vorherigen Ansprüche
**dadurch gekennzeichnet, dass**
eine Größe der Behandlungsfläche zwischen 30 mm² und 50 mm² oder zwischen 6 cm² und 18 cm² beträgt.
